# EUROPEAN PATENT SPECIFICATION

(11) **EP 3 534 793 B1**
(45) Date of publication and mention of the grant of the patent: **31.12.2025**
(21) Application number: 17875181.4
(22) Date of filing: 03.11.2017
(51) Int. Cl.: A61B 5/145, A61M 5/142

(54) **METHODS, SYSTEMS, AND IMPLANTABLE DEVICES FOR ENHANCING BLOOD GLUCOSE REGULATION**
VERFAHREN, SYSTEME UND IMPLANTIERBARE VORRICHTUNGEN ZUR VERBESSERTEN REGULIERUNG DES BLUTZUCKERSPIEGELS
PROCÉDÉS, SYSTÈMES ET DISPOSITIFS IMPLANTABLES POUR AMÉLIORER LA RÉGULATION DE LA GLYCÉMIE

(30) Priority: 03.11.2016 US 201662417060 P
(43) Date of publication of application: 11.09.2019
(73) Proprietor: Arizona Board of Regents on behalf of the University of Arizona, Tucson, AZ 85721 (US)
(72) Inventor: PAPAS, Klearchos, K., Tucson AZ 85724 (US)
(74) Representative: J A Kemp LLP
(86) International application number: PCT/US2017/060043
(87) International publication number: WO 2018/102077

(56) References cited:
- US-A- 5 169 390
- US-A- 5 324 518
- US-A- 5 324 518
- US-A- 5 368 028
- US-A- 5 741 330
- US-A1- 2003 087 427
- US-A1- 2004 197 374
- US-A1- 2010 228 110
- US-A1- 2013 289 540
- US-A1- 2015 112 247

## Description

### FIELD OF THE INVENTION

The present invention relates to a system for regulating blood glucose, more particularly to implantable encapsulated devices with glucose sensors, optionally with insulin secreting cells and insulin infusion systems.

### BACKGROUND OF THE INVENTION

The present invention relates to systems for regulating blood glucose, such as implantable encapsulated devices with glucose sensors optionally with insulin secreting cells and insulin infusion systems. For example, the present invention may feature encapsulation devices (with a glucose sensors) connected to an insulin infusion pump for distribution of insulin. The insulin infusion pump may feature an insulin pouch fluidly connected to an insulin pump. In some embodiments not according to the invention, a glucose sensor is separate from the encapsulation device. The system may feature an additional implantable device comprising insulin secreting cells.

The disclosures of the following U.S. Patents referred to herein: U.S. Pat. No. 5,713,888; U.S. Pat. App. No. 2003/0087427. Relevant prior art can further be found under US 5,324,518; US 2015/0112247A1; and US 2013/0289540A1.

### SUMMARY OF THE INVENTION

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes. The present invention features a system for regulating blood glucose. For example, the present invention features a system comprising a vascularized encapsulation device and a glucose sensor disposed therein. In some embodiments, the encapsulation device is operatively connected to an insulin infusion pump for distribution of insulin. In some embodiments, the system further comprises a gas channel disposed adjacent to the encapsulation device. in some embodiments, the system further comprises a gas channel disposed within the encapsulation device.

In some embodiments, the encapsulation device is free of cells. In some embodiments, the encapsulation device comprises cells. In some embodiments, the encapsulation device is vascularized.

In some embodiments, the insulin infusion pump comprises an insulin pouch fluidly connected to an insulin pump.

In some embodiments, the system further comprises a second encapsulation device comprising insulin secreting cells, glucagon secreting cells, or a combination thereof. In some embodiments, the cells (e.g., glucagon secreting cells) help prevent hypoglycemia.

In some embodiments, the glucose sensor is operatively connected to the insulin infusion pump via a closed loop controller, wherein when the glucose sensor detects a level of glucose that is at or above a threshold level of glucose, the glucose sensor sends a signal to the closed loop controller, whereupon the closed loop controller sends a signal to the insulin pump to release an amount of insulin. In some embodiments, the system is adapted to adjust insulin secretion based on glucose levels measured by the glucose sensor.

In some embodiments, the encapsulation device comprises insulin secreting cells, glucagon secreting cells, or a combination thereof. In some embodiments, the encapsulation device is pre-vascularized prior to loading islets into the encapsulation device.

In some embodiments, the glucose sensor is wirelessly connected to a system adapted to relay glucose levels detected by the glucose sensor. In some embodiments, the glucose sensor is replaceable.

The encapsulation devices of the present invention feature vascularization (vascularization helps the glucose sensor work).

Glucagon secreting cells may help sense hypoglycemia.

In some embodiments, the device has more than one sensor and/or has one or more sensor types. Having different sensor types may offer advantages, e.g., the ability to combine the readings, e.g., if one goes up, one goes down, etc.

In some embodiments, the encapsulation device containing the glucose sensors and one used from insulin delivery are separate devices and in separate locations (e.g., separate arms or arm versus abdomen). This separation may also be useful for a device containing insulin secreting and glucagon secreting cells (human islets have both insulin secreting and glucagon secreting cells within them). In some embodiments, it is at a distance from the device containing glucose sensors and the device containing insulin secreting cells.

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description and claims.

### BRIEF DESCRIPTION OF THE DRAWINGS

The features and advantages of the present invention will become apparent from a consideration of the following detailed description presented in connection with the accompanying drawings in which:
FIG. 1A shows an example of a single-chamber encapsulation device for holding cells or tissues. The device comprises a port to access the lumen for loading the cells or tissue.
FIG. 1B shows a cross-sectional view of the device of FIG. 1A. The cells are encapsulated in a two-layer membrane envelope formed using a mesh insert. The device comprises a vascularization membrane and an immunoisolation membrane. The present invention is not limited to devices that utilize an immunoisolation membrane: in some embodiments, the device only comprises the vascularization membrane.
FIG. 2A shows a detailed view of an encapsulation device with an immunoisolation membrane. The device features two chambers or lumens separated by a gas channel.
FIG. 2B shows a detailed view of an encapsulation device without the immunoisolation membrane. The device features two chambers or lumens separated by a gas channel.
FIG. 3 shows a schematic view of an encapsulation device comprising a glucose sensor disposed therein.
FIG. 4A shows a schematic view of a system of the present invention comprising an encapsulation device with a first sensor disposed therein and a second sensor.
FIG. 4B shows an optical reader for reading sensors in an encapsulation device.
FIG. 4C shows a sensor reader, e.g., an electrochemical glucose (or oxygen or lactate) sensor reader (via a wire going through the skin), which is an alternative to having some of the electronic components fully implanted.
FIG. 5A shows a schematic view of a system of the present invention comprising a single-chamber encapsulation device with a glucose sensor disposed therein and an oxygen delivery channel (for oxygen or air) disposed adjacent to the device. Vasculature is present.
FIG. 5B shows a schematic view of a system of the present invention comprising a dual-chamber encapsulation device with a sensor disposed in each device. An oxygen delivery channel (for oxygen or air) is disposed between the two chambers of the device. Vasculature is present.

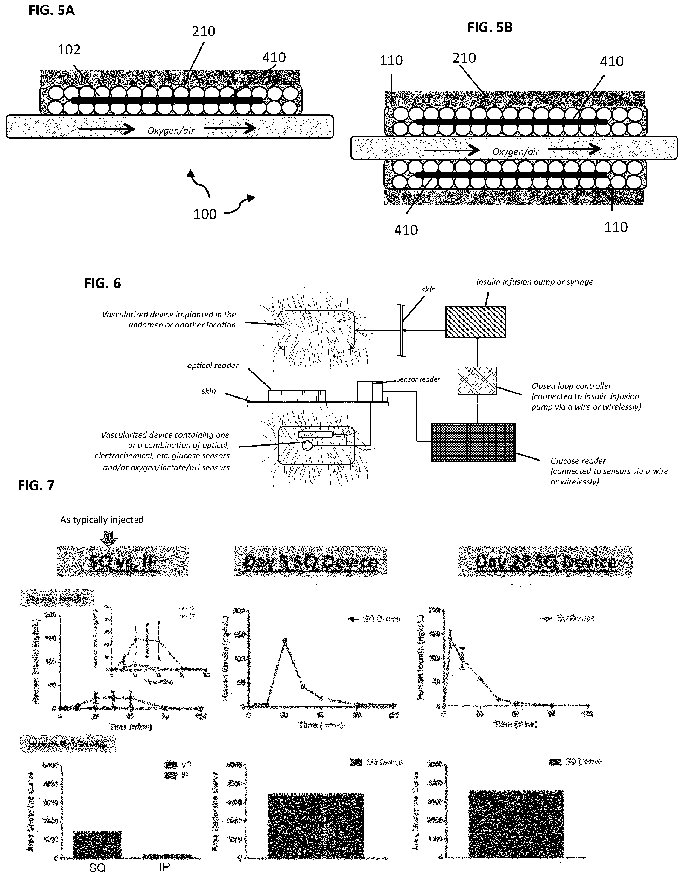

FIG. 6 shows schematic of the components of various embodiments of the present invention. A vascularized device is implanted (e.g., in the abdomen or other location), which is connected to an insulin infusion pump or syringe, which is connected to a closed-loop controller (may be connected to an insulin infusion pump either via a wire or wirelessly), which is connected to a glucose reader (connected to sensors wirelessly or via a wire), which is connected to a sensor reader, which reads a sensor in an implanted device (a vascularized device containing one or a combination of optical, electrochemical sensors, a glucose sensor and/or an oxygen sensor, lactate sensor, pH sensor, etc. Further, FIG. 6 shows a non-limiting example of a system of the present invention, e.g., an insulin regulating system. The present invention is not limited to the configurations and components shown in FIG. 6. In some embodiments, the insulin regulation system of the present invention comprises an encapsulation device operatively connected to an insulin infusion pump (or can be connected to a syringe for insulin injection on an as needed basis) for distribution of insulin. In some embodiments, the encapsulation device is free of cells. In some embodiments, the encapsulation device comprises cells. The encapsulation device may be vascularized; however the present invention is not limited to vascularized encapsulation devices. In some embodiments, the vascularized device connected to the insulin infusion pump or the insulin injection syringe does not contain any sensors. In some other cases it contains an oxygen sensor or a combination of glucose sensor, or a lactate sensor or a combination of all sensors. In some cases, the device containing glucose sensors which communicate through a glucose reader with the closed loop controller contains multiple glucose sensors including optical as well as electrochemical oxygen sensors. In some cases the device containing insulin and glucagon secreting cells is located near the device connected to an insulin infusion pump and the device containing the separate device glucose and other sensors and in some cases each of the devices is located far apart. In some cases, multiple or more than one devices containing cells or containing sensors or connected to insulin infusion systems are implanted within one person and in some cases these devices are placed far apart from each other.
FIG. 7 shows data demonstrating better insulin release kinetics in a rat when insulin was infused through the port of an implanted device after vasculature formed around it (e.g., at 28 days post-transplant). The peak of insulin appearance in the blood shifts to the left (faster) on day 28 (presumably because of better/more vasculature around the device) and also much better total insulin in blood (AUC) when insulin injected through the device (both 5 as well as 28 days post-device implantation). The same amount of insulin was infused through the device subQ (SQ) 5 days and 28 days post implantation.

### DETAILED DESCRIPTION OF THE INVENTION

### Encapsulation Devices

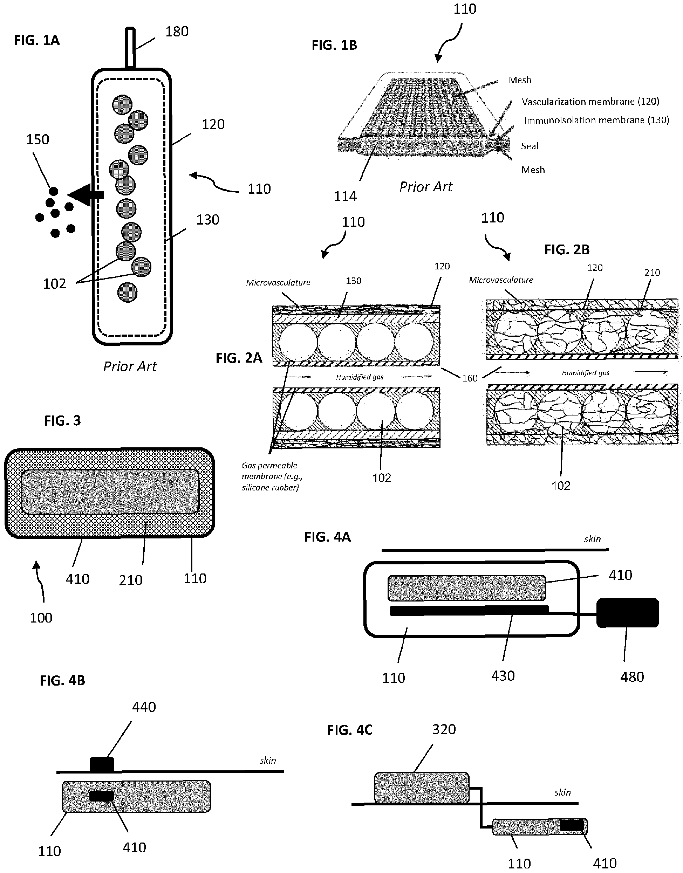

Encapsulation devices are devices for holding cells or tissues, but they can also hold sensors, particles for slow release of therapeutic agents, etc., for example. The encapsulation device (110) shown in FIG. 1A is a single-chamber encapsulation device. The device (100) comprises an inner lumen for holding the cells (102) or tissue and at least one membrane, e.g., a vascularization membrane (120), which is impermeable to cells. In some embodiments, the device (100) further comprises an immunoisolation membrane (130). Non-cell factors or molecules (150) can escape the cell impermeable membrane. The device (110) also comprises a port (180) to access the lumen for loading the cells or tissue. FIG. 1B shows a cross-sectional view of an encapsulation device. The cells are encapsulated in a lumen (114) by a two-layer membrane envelope, a vascularization membrane (120) and an immunoisolation membrane (130). The device (110) also has structural support, e.g., mesh, seals, etc.

In some embodiments, the encapsulation devices (110) comprise a vascularization membrane (120) and immunoisolation membrane (130). In some embodiments, the encapsulation devices (110) comprise just the vascularization membrane (120). This allows blood vessels to grow within the transplanted tissue.

In the examples shown in FIG. 1A and FIG. 1B, the cells therein are about 5-15 µm in diameter. The outer membrane, the vascularization membrane (120), has a pore size from 5-10 µm. The vascularization membrane (120) is about 15 µm thick. The immunoisolation membrane (130) has a pore size of about 0.4 µm. The immunoisolation membrane (130) is about 30 µm thick. In some embodiments, the membranes (120, 130) are constructed from materials such as polytetraflouroethylene (PTFE) or other similar material. The present invention is not limited to the aforementioned pore sizes and thicknesses of the membranes used therein. The present invention is not limited to the aforementioned materials.

The encapsulation devices (110) may be constructed in various shapes and sizes and with various lumen volumes. For example, in some embodiments, the lumen has a volume of about 4.5 µl. In some embodiments, the lumen has a volume of 20 µl. In some embodiments, the lumen has a volume of 40 µl. In some embodiments, the device (110) is from 4 to 5 cm in length. In some embodiments, the device (110) is from 2 to 5 cm in length, e.g., 3 cm. In some embodiments, the device (110) is from 5 to 10 cm in length. The present invention is not limited to the aforementioned dimensions and lumen volumes. For example, in some embodiments, the lumen has a volume of about 100 µl. In some embodiments, the lumen has a volume of about 200 µl. In some embodiments, the lumen has a volume from 2 to 50 µl. In some embodiments, the lumen has a volume from 10 to 100 µl. In some embodiments, the lumen has a volume from 40 to 200 µl. In some embodiments, the lumen has a volume from 100 to 300 µl. In some embodiments, the lumen has a volume from 200 to 500 µl.

In some embodiments, within the encapsulation devices (110), there may be layers of cells or tissue, e.g., multiple lumens within the device (110). For example, an encapsulation device (110) may comprise two chambers or lumens. In some embodiments, the device comprises more than two chambers or lumens, e.g., 3 chambers or lumens, 4 chambers or lumens, 5 chambers or lumens, etc. FIG. 2A and FIG. 2B show examples an encapsulation with two lumens (two chambers) that are separated by a gas channel (160). FIG. 2A and FIG. 2B also show vascularizing membrane and microvasculature. The blood vessels embed into the vascularizing membrane.

In some embodiments, the chamber or lumen comprises a single layer of cells. In some embodiments, the chamber or lumen comprises two layers of cells. In some embodiments, the chamber comprises three or more layers of cells. In some embodiments, islet spheroids (about 150 um in size) are used (shown in FIG. 2A, FIG. 2B). In some embodiments, a dual layer of the islet spheroids is used (lumen thickness would be about 300 um in the chamber or in each chamber). In some embodiments, a third layer is supported depending on the metabolic activity and other characteristics of the spheroids/cells used. Note spheroids may not be touching each other in some configurations and the space between them may be 1 or 2 spheroids apart (e.g., 150 um, 300 um), or more or less.

### Methods and Systems for Regulating Blood Glucose

The present invention features a system for regulating blood glucose. The system may comprise an encapsulated device (110) and one or more glucose sensors (410). The system further comprises oxygen delivery. Oxygen may be delivered via several mechanisms, e.g., an air pump, an oxygen generator, etc. Without wishing to limit the present invention to any theory or mechanism, it is believed that oxygen or air delivery is important for the glucose sensors because of the enzyme glucose oxidase. Oxygen (or air) delivery will allow more of the enzyme (e.g. glucose oxidase) to be incorporated within the sensor as the chemical reaction enabling glucose measurements with this enzyme will no longer be oxygen limited. The oxygen or air delivery may allow for more accurate readings as well as the extension in the life of the sensor system. By incorporating the sensors within the vascularizing encapsulation device with enhanced oxygen delivery, in addition to minimizing biofouling and improving glucose sensing kinetics, the signal to noise and longevity of the sensors could also be increased by enabling more enzyme to be used.

The glucose sensor or glucose sensors may cover the interior surface of the encapsulation device, e.g., the entire interior surface that is vascularized. FIG. 3 shows an example of a glucose sensor disposed in an encapsulation device. The device may be vascularized (210).

The sensors may be optical, electrochemical, nuclear magnetic resonance (NMR)-based, or a combination thereof. For example, there may be a combination of sensors in the same device.

FIG. 4A shows a schematic view of a system of the present invention comprising an vascularizing encapsulation device (110) with an optical glucose sensor (410) disposed therein communicating to an optical reader (e.g., implanted on top of the device, separated enough from ten device incorporating the sensor so that vasculature is available around the device containing the sensor for proper glucose sensing and kinetics) and an electrochemical sensor (430). The electrochemical sensor (430) is operatively connected to an electronic signal component (480) that can send a wireless signal to a particular receiving component (e.g., cell phone or other piece of equipment). The electronics may be flexible, sealed, and/or encapsulated and can be fully implanted of if necessary connected through the skin and be outside the body. An optical reader may be either above the skin or below if it is implanted. FIG. 4B shows an optical reader (440) for reading the sensors (401). FIG. 4C shows a sensor reader (320), e.g., an electrochemical glucose (or oxygen or lactate) sensor reader (via a wire going through the skin), which is an alternative to having some of the electronic components fully implanted as shown in FIG. 4A and having some of them connected through the skin to the outside of the body.

FIG. 5A shows a schematic view of a system of the present invention comprising a single-chamber encapsulation device with a glucose sensor disposed therein and an oxygen delivery channel (for oxygen or air) disposed adjacent to the device. Vasculature is present.

FIG. 5B shows a schematic view of a system of the present invention comprising a dual-chamber encapsulation device with a sensor disposed in each device. An oxygen delivery channel (for oxygen or air) is disposed between the two chambers of the device. Vasculature is present.

FIG. 6 shows a non-limiting example of a system of the present invention, e.g., an insulin regulating system. The present invention is not limited to the configurations and components shown in FIG. 6. In some embodiments, the insulin regulation system of the present invention comprises an encapsulation device operatively connected to an insulin infusion pump (or can be connected to a syringe for insulin injection on an as needed basis) for distribution of insulin. In some embodiments, the encapsulation device is free of cells. In some embodiments, the encapsulation device comprises cells. The encapsulation device may be vascularized; however the present invention is not limited to vascularized encapsulation devices.

In some embodiments, the system of the present invention comprises an encapsulation device with a glucose sensor. In some embodiments, the system comprises an implanted encapsulation device with insulin/glucagon secreting cells. In some embodiments, the system comprises an implanted encapsulation device connected to an insulin infusion pump. In some embodiments, the system of the present invention comprises an encapsulation device with a glucose sensor, and/or insulin/glucagon secreting cells and/or an insulin infusion pump.

In some embodiments, the glucose sensor is operatively connected to a cell phone or other system that can receive signals that reflect the glucose levels. For example, the glucose sensor may be wirelessly connected to a system adapted to relay glucose levels detected by the glucose sensor. In some embodiments, a wireless system is operatively connected to the insulin infusion pump to allow remote regulation of the insulin infusion pump.

In some embodiments, the encapsulation device comprises an immunoisolation membrane. In some embodiments, the encapsulation device does not comprise an immunoisolation membrane.

In some embodiments, the insulin infusion pump comprises an insulin pouch fluidly connected to an insulin pump. In some embodiments, the system comprises a glucose sensor separate from the encapsulation device. In some embodiments, the glucose sensor is housed in a separate implantable device. In some embodiments, the system comprises an additional implantable device comprising insulin secreting cells (e.g., for helping to prevent hypoglycemia).

In some embodiments, the glucose sensor is operatively connected to the insulin infusion pump via a closed loop controller, wherein when the glucose sensor detects a level of glucose that is at or above a threshold level of glucose, the glucose sensor sends a signal to the closed loop controller, whereupon the closed loop controller sends a signal to the insulin pump to release an amount of insulin.

The system of the present invention may be adapted to adjust insulin secretion based on glucose levels measured by the glucose sensor. In some embodiments, the encapsulation device further comprises insulin secreting cells. In some embodiments, the encapsulation device is pre-vascularized prior to loading islets and extracellular matrix. In some embodiments, the glucose sensor is replaceable without explanting the device.

FIG. 7 shows better insulin release kinetics in a rat when insulin was infused through the port of an implanted device after vasculature formed around it (definitely at 28 days post-transplant. Infusion through a port and into a vascularized device may be much better. In some embodiments, a glucose sensor is disposed in a device that is vascularized, and the sensor may help improve the longevity, integration with the body and kinetics of glucose sensing. If a glucose sensor in a device could be connected with a controller to an insulin infusion pump delivering insulin through a device (at a different location in the body) then this artificial pancreas may be better as compared to existing state-of-the art systems and a lot more cost effective. In some embodiments, a device is implanted in yet another location (e.g., this device without exogenous oxygen delivery) (the device could be stacked devices and/or prevascularized, may contain an oxygen sensor to indicate when adequate vascularization is established, etc.). Insulin secreting cells and glucagon secreting cells (human islets, stem cell derived islets, etc.) may then be implanted in the device and release insulin and glucagon in response to glucose fluctuations and they will stabilize blood glucose levels and will reduced hypoglycemic episodes. They may provide the majority or all insulin needed in a patient and additional insulin needed may be supplemented by a pump or injections.

The present invention features encapsulation devices operatively connected to an insulin infusion pump for distribution of insulin. In some embodiments, the encapsulation device is free of cells. In some embodiments, the encapsulation device comprises cells. In some embodiments, the encapsulation device is vascularized. In some embodiments, the encapsulation device comprises an immunoisolation membrane. In some embodiments, the encapsulation device does not comprise an immunoisolation membrane. In some embodiments, the insulin infusion pump comprises an insulin pouch fluidly connected to an insulin pump. In some embodiments, the system comprises a glucose sensor separate from the encapsulation device. In some embodiments, the glucose sensor is housed in a separate implantable device. In some embodiments, comprising an additional implantable device comprising insulin secreting cells. In some embodiments, the cells help prevent hypoglycemia. In some embodiments, the glucose sensor is operatively connected to the insulin infusion pump via a closed loop controller, wherein when the glucose sensor detects a level of glucose that is at or above a threshold level of glucose, the glucose sensor sends a signal to the closed loop controller, whereupon the closed loop controller sends a signal to the insulin pump to release an amount of insulin. In some embodiments, the system is adapted to adjust insulin secretion based on glucose levels measured by the glucose sensor. In some embodiments, the encapsulation device further comprises insulin secreting cells. In some embodiments, the encapsulation device is pre-vascularized prior to loading islets and extracellular matrix. In some embodiments, the glucose sensor is wirelessly connected to a system adapted to relay glucose levels detected by the glucose sensor. In some embodiments, the glucose sensor is replaceable.

In some embodiments, the devices of the systems of the present invention are temporarily oxygenated. For example, in some embodiments, oxygen is temporarily delivered initially (e.g., initially upon implantation) until the system is adequately vascularized. In some embodiments, oxygen may be temporarily delivered and/or oxygen levels may be variable. For example, in some embodiments, a cell type is used that benefits from a high oxygen level. In some embodiments, a cell type is used that benefits from a low oxygen level (e.g., 15% or lower). In some embodiments, an oxygen level of about 21% oxygen (e.g., 20-22%) is used, e.g., air may be used. In some embodiments, an oxygen level from 15-22% is used. In some embodiments, an oxygen level from 10-15% is used. In some embodiments, an oxygen level from 5-10% is used. In some embodiments, an oxygen level from 0-5% is used. In some embodiments, a particular oxygen level is used initially and then the oxygen level is increased or decreased at a later time. In some embodiments, oxygen is turned on and then off. In some embodiments, oxygen is turned off and then on. In some embodiments, oxygen is turned on and off in a cycle for a period of time or indefinitely. In some embodiments, oxygen level is tailored to the application to help modulate the local immune system by providing temporary oxygen. In some embodiments, oxygen levels are tailed to when vascularization occurs. In some embodiments, immature cells are transplanted, and low oxygen levels may be used initially; as the cells mature (e.g., after a particular time, e.g., 4-6 weeks), higher oxygen levels may be provided.

Oxygen may be delivered to the systems via several different mechanisms. For example, the system of the present invention may comprise an oxygen generator or an air pump. In some embodiments, the oxygen generator is an implantable oxygen generator, which is well known to one of ordinary skill in the art. For example, the implantable oxygen generator may feature an electrochemical oxygen generation mechanism (e.g., using electricity to break down water to oxygen hydrogen), a chemical mechanism, or other mechanism. In some embodiments, the oxygen generator is a wearable oxygen generator or pump. In some embodiments, the oxygen is delivered via a carrier media like hemoglobin or fluorinated microbubbles. The present invention is not limited to the aforementioned systems or materials.

Various modifications of the invention, in addition to those described herein, will be apparent to those skilled in the art from the foregoing description. Such modifications are also intended to fall within the scope of the appended claims..

Although there has been shown and described the preferred embodiment of the present invention, it will be readily apparent to those skilled in the art that modifications may be made thereto which do not exceed the scope of the appended claims. Therefore, the scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of', and as such the written description requirement for claiming one or more embodiments of the present invention using the phrase "consisting of' is met.

The reference numbers recited in the below claims are solely for ease of examination of this patent application, and are exemplary, and are not intended in any way to limit the scope of the claims to the particular features having the corresponding reference numbers in the drawings.

## Claims

1. A system for regulating blood glucose, the system comprising:
an implantable encapsulation device (110) wherein the device is from 2 to 10 centimeters in length, and comprises:
a lumen (114) for holding cells or tissue, wherein the lumen has a volume from 2 to 100 µl; and
a vascularization membrane (120) around said lumen, wherein the vascularization membrane has a pore size from 5 to 10µm;
a gas channel (160) disposed within, or adjacent to, the encapsulation device; and
a glucose sensor (410) disposed within said encapsulation device;
wherein the glucose sensor is configured to be connected to a system (320; 440; 430, 480) adapted to relay glucose levels detected by the glucose sensor.

2. The system of claim 1, wherein the encapsulation device is operatively connected to an insulin infusion pump or syringe for distribution of insulin.

3. The system of claim 1 or 2, wherein said gas channel is disposed adjacent to the encapsulation device.

4. The system of claim 1 or 2, wherein said gas channel is disposed within the encapsulation device.

5. The system of any of claims 1-4, wherein the glucose sensor is optical or electrochemical.

6. The system of any of claims 1-4, wherein the encapsulation device comprises cells.

7. The system of any of claims 1-6, wherein the encapsulation device is vascularized.

8. The system of claim 2, wherein the insulin infusion pump comprises an insulin pouch fluidly connected to an insulin pump.

9. The system of claim 6, wherein said cells are insulin secreting cells, glucagon secreting cells, or a combination thereof.

10. The system of claim 9, wherein the glucagon secreting cells help prevent hypoglycemia.

11. **The** system of claim 2 or 8, wherein the glucose sensor is operatively connected to the insulin infusion pump or syringe via a closed loop controller, wherein when the glucose sensor detects a level of glucose that is at or above a threshold level of glucose, the glucose sensor sends a signal to the closed loop controller, whereupon the closed loop controller sends a signal to the insulin pump or syringe to release an amount of insulin.

12. **The** system of any of claims 2-11, wherein the system is adapted to adjust insulin secretion based on glucose levels measured by the glucose sensor.

13. **The** system of 1-5, wherein the encapsulation device is pre-vascularized prior to loading islets into the encapsulation device.

14. **The** system of claim 1, wherein the glucose sensor is wirelessly connected to said system adapted to relay glucose levels detected by the glucose sensor.

15. **The** system of claim 1, wherein the glucose sensor is replaceable.

## Patentansprüche

1. System zur Regulierung von Blutzucker, das System umfassend:
eine implantierbare Einkapselungsvorrichtung (110), wobei die Vorrichtung eine Länge von 2 bis 10 Zentimetern aufweist und Folgendes umfasst:
ein Lumen (114) zum Halten von Zellen oder Gewebe, wobei das Lumen ein Volumen von 2 bis 100 µl aufweist; und
eine Vaskularisationsmembran (120) um das Lumen herum, wobei die Vaskularisationsmembran eine Porengröße von 5 bis 10 µm aufweist;
einen Gaskanal (160), der innerhalb oder angrenzend an die Einkapselungsvorrichtung angeordnet ist; und einen Glucosesensor (410), der innerhalb der Einkapselungsvorrichtung angeordnet ist;
wobei der Glucosesensor konfiguriert ist, mit einem System (320; 440; 430, 480) verbunden zu werden, das angepasst ist, von dem Glucosesensor erfasste Blutzuckerspiegel weiterzugeben.

2. System nach Anspruch 1, wobei die Einkapselungsvorrichtung wirkend mit einer Insulininfusionspumpe oder -spritze zur Abgabe von Insulin verbunden ist.

3. System nach Anspruch 1 oder 2, wobei der Gaskanal angrenzend an die Einkapselungsvorrichtung angeordnet ist.

4. System nach Anspruch 1 oder 2, wobei der Gaskanal innerhalb der Einkapselungsvorrichtung angeordnet ist.

5. System nach einem der Ansprüche 1 bis 4, wobei der Glucosesensor ein optischer oder elektrochemischer Sensor ist.

6. System nach einem der Ansprüche 1 bis 4, wobei die Einkapselungsvorrichtung Zellen umfasst.

7. System nach einem der Ansprüche 1 bis 6, wobei die Einkapselungsvorrichtung vaskularisiert ist.

8. System nach Anspruch 2, wobei die Insulininfusionspumpe einen Insulin-Beutel umfasst, der fluidisch mit einer Insulinpumpe verbunden ist.

9. System nach Anspruch 6, wobei die Zellen Insulin sekretierende Zellen, Glukagon sekretierende Zellen oder eine Kombination davon sind.

10. System nach Anspruch 9, wobei die Glukagon sekretierenden Zellen dabei helfen, Hypoglykämie zu verhindern.

11. System nach Anspruch 2 oder 8, wobei der Glucosesensor wirkend mit der Insulininfusionspumpe oder -spritze über einen geschlossenen Regler verbunden ist, wobei, wenn der Glucosesensor einen Blutzuckerspiegel erfasst, der auf oder über einem Schwellenwert des Blutzuckerspiegels liegt, der Glucosesensor ein Signal an den geschlossenen Regler sendet, worauf der geschlossene Regler ein Signal an die Insulinpumpe oder -spritze sendet, um eine Insulinmenge abzugeben.

12. System nach einem der Ansprüche 2 bis 11, wobei das System so angepasst ist, dass es eine Insulinsekretion basierend auf den Blutzuckerspiegeln, die von dem Glucosesensor gemessen werden, einstellt.

13. System nach einem der Ansprüche 1 bis 5, wobei die Einkapselungsvorrichtung vorvaskularisiert wird, bevor Inseln in die Einkapselungsvorrichtung geladen werden.

14. System nach Anspruch 1, wobei der Glucosesensor drahtlos mit dem System verbunden ist, das so angepasst ist, dass es die von dem Glucosesensor erfassten Blutzuckerspiegel weiterleitet.

15. System nach Anspruch 1, wobei der Glucosesensor austauschbar ist.

## Revendications

1. Système pour réguler la glycémie, le système comprenant :
un dispositif d'encapsulation implantable (110), dans lequel le dispositif mesure de 2 à 10 centimètres de long, et comprend :
une lumière (114) destinée à contenir des cellules ou un tissu, dans lequel la lumière a un volume de 2 à 100 µl ; et
une membrane de vascularisation (120) autour de ladite lumière, dans lequel la membrane de vascularisation a une taille des pores de 5 à 10 µm ;
un canal de gaz (160) disposé à l'intérieur du dispositif d'encapsulation, ou de façon adjacente à celui-ci ; et
un capteur de glucose (410) disposé à l'intérieur dudit dispositif d'encapsulation ;
dans lequel le capteur de glucose est configuré pour être raccordé à un système (320 ; 440 ; 430, 480) adapté pour relayer des niveaux de glucose détectés par le capteur de glucose.

2. Système de la revendication 1, dans lequel le dispositif d'encapsulation est raccordé opérationnellement à une pompe ou seringue de perfusion d'insuline pour distribution d'insuline.

3. Système de la revendication 1 ou 2, dans lequel ledit canal de gaz est disposé de façon adjacente au dispositif d'encapsulation.

4. Système de la revendication 1 ou 2, dans lequel ledit canal de gaz est disposé à l'intérieur du dispositif d'encapsulation.

5. Système de quelconques des revendications 1 à 4, dans lequel le capteur de glucose est optique ou électrochimique.

6. Système de quelconques des revendications 1 à 4, dans lequel le dispositif d'encapsulation comprend des cellules.

7. Système de quelconques des revendications 1 à 6, dans lequel le dispositif d'encapsulation est vascularisé.

8. Système de la revendication 2, dans lequel la pompe de perfusion d'insuline comprend un sachet d'insuline raccordé fluidiquement à une pompe à insuline.

9. Système de la revendication 6, dans lequel lesdites cellules sont des cellules sécrétrices d'insuline, des cellules sécrétrices de glucagon, ou une combinaison de celles-ci.

10. Système de la revendication 9, dans lequel les cellules sécrétrices de glucagon aident à empêcher l'hypoglycémie.

11. Système de la revendication 2 ou 8, dans lequel le capteur de glucose est raccordé opérationnellement à la pompe ou seringue de perfusion d'insuline par l'intermédiaire d'une unité de commande en boucle fermée, dans lequel, lorsque le capteur de glucose détecte un niveau de glucose qui est à un niveau seuil de glucose ou supérieur à celui-ci, le capteur de glucose envoie un signal à l'unité de commande en boucle fermée, après quoi l'unité de commande en boucle fermée envoie un signal à la pompe ou seringue à insuline pour libérer une quantité d'insuline.

12. Système de quelconques des revendications 2 à 11, dans lequel le système est adapté pour régler une sécrétion d'insuline sur la base de niveaux de glucose mesurés par le capteur de glucose.

13. Système des revendications 1 à 5, dans lequel le dispositif d'encapsulation est prévascularisé avant de charger des îlots dans le dispositif d'encapsulation.

14. Système de la revendication 1, dans lequel le capteur de glucose est raccordé sans fil audit système adapté pour relayer des niveaux de glucose détectés par le capteur de glucose.

15. Système de la revendication 1, dans lequel le capteur de glucose est remplaçable.
